# EUROPEAN PATENT APPLICATION

(11) **EP 2 371 407 A1**
(43) Date of publication of application: **05.10.2011**
(21) Application number: 10194868.5
(22) Date of filing: 14.12.2010
(51) Int. Cl.: A61M 5/32, A61M 5/50

(54) **Syringe having a retractable needle**

(30) Priority: 31.03.2010 AU 2010901349
(71) Applicant: Just Innovative Layouts Pty Ltd., Slacks Creek QLD 4127 (AU)
(72) Inventor: Leigh Kiehne, Bruce, Slacks Creek, Queensland 4127 (AU)
(74) Representative: Modiano, Micaela Nadia

(57) **Abstract**

A syringe (100) comprising: a barrel (1), a needle holder (12) that holds a hypodermic needle (14) through a needle opening (102), and a hollow plunger (6), wherein the needle holder (12) is biased away from the needle opening (102) such that when the hollow plunger (6) moves through the injection stroke the needle holder (12) is propelled by the biasing member (13) to retract into the hollow plunger (6).

## Description

### FIELD OF THE INVENTION

The present invention relates generally to medical instruments and more particularly to a syringe having a retractable needle and designed for single use and disposal after use.

### BACKGROUND OF THE INVENTION

Healthcare workers routinely risk exposure to communicable diseases through accidental exposure to contaminated medical products, waste products and bodily fluids. One of the largest exposure risks to healthcare workers and handlers of related hazardous waste is from accidental needlesticks or scratches while using hypodermic syringes. The Centers for Disease Control and Prevention in the United States of America reports that there are in excess 20 of five hundred thousand reported accidental needlesticks each year and an estimated three million additional needlesticks not reported. It is further reported that the odds of a healthcare worker contracting human immunodeficiency virus (HIV) alone through a needlestick are one in 300. The odds of a healthcare worker contracting any of many other serious or potentially fatal diseases such as hepatitis are even greater. In response to this health issue some US states and the US federal government are enacting legislation requiring exclusive use of safety hypodermic syringes with retractable or protected needles, for example, by the Federal Needlestick Safety and Prevention Act.

Another source of cross contamination infection has come to light in present years is dead or waste space. This is the amount of the residual fluid or medication left in the syringe after the injection stroke and the safety mechanism has been activated. The International Organization for Standardization or more commonly known as I.S.O have a standard procedure written to measure the residual fluid left in a syringe after it has been used. While every syringe has some amount of residual fluid, some brands of syringe have more than others and it is important to know how much residual fluid is in each particular brand so that they may be calibrated in order to deliver an accurate dose of medication.

This may seem like a waste of medication but the real problem is when a spring loaded retractable syringe or similar is used, the sudden shock of retraction can cause the residual fluid to spray or squirt out of the front of he barrel during the retraction process, and this is what is known in the industry as 'splatter'. The splattering effect causes a number of problems. The spraying of fluids, which could be contaminated, around the operating or emergency rooms is highly undesirable, and even worse this fluid could become atomized in the air and be breathed in causing cross contamination, the very thing the retractable syringes were designed to prevent.

Other problems faced today with safety medical devices are complexity of design and manner of operation. While there are hundreds of designs for safety syringes around the world very few make it to the market. This is mainly because of their complexity in design which makes them very difficult and sometimes impossible to mass produce therefore making them unviable. The few that have made it to the market often have a lot more moving parts than a standard syringe and this makes them sometimes up to four times more expensive than a standard syringe, this cost making them unaffordable to many. Furthermore, because they have more parts there is a higher chance of them failing.

In most cases these safety devices require the user to manipulate the device in some way after the injection stroke either by pulling a sheath down to cover the needle or by pulling the plunger rod back up the barrel and the needle with it. Others need the user to push the plunger rod further forward after the injection stroke in order for the retraction mechanism to work and in a lot of cases these designs start to engage their various retraction mechanisms well before the injection stroke has finished. This destabilizes their needle holders and sealing arrangement and by hydraulic dynamics that can cause premature activation and leaking where hydraulic forces are generated by depressing the plunger rod and forcing the fluid in the variable fluid chamber into the smaller diameter of the needle holder fluid path. Others also appear to have a lack of mechanical advantage over their retraction mechanisms so that they would require a significant amount of force to activate. Often in emergency and operating rooms the user has no time to manipulate these devices so that they go inactivated or the user becomes bewildered by seeing parts of the retraction mechanisms still left in the barrel after activation and they are unsure whether the device has failed in some way or not.

US6090077 (Shaw) describes a retractable syringe that has a retaining ring that holds the needle holder in a non retracting position by frictional or a hooping clamping force. An alternate embodiment of the invention has no retaining ring and the needle holder is held in a non-retracting position by frictional force or a hooping force by the barrel itself.

The alternate embodiment describes having a tiny ramp provided at the transition zone of the barrel where when the head of the plunger rod is pushed in a downward motion the head of the plunger rod spreads or forces the barrel outwardly while dislodging the stopper that seals the front of the retraction cavity, thereby reducing the clamping or friction force on the head of the needle holder provided by the wall of the barrel. The holding force is thereby reduced to below the retraction force provided by the compressed spring and the needle holder is ejected into the retraction cavity of the plunger and carries the dislodged stopper along with it.

There are a number of problems faced with this design. Any parts that are frictionally engaged are prone to tight tolerances control at the point at manufacture and are temperature sensitive. Also, because no parts are broken or destroyed during or after retraction it is easily put back together and reused. There is furthermore an amount of dead or waste space at the point of retraction.

What is needed is a safety device that addresses all of the above and activates automatically after as much fluid or medication as possible has been expelled.

### SUMMARY OF THE INVENTION

In accordance with the present invention there is provided a syringe comprising: a barrel; a needle holder positioned inside the barrel that holds a hypodermic needle through a needle opening in the barrel; and a hollow plunger sealingly slidable within the barrel and having a deformable member across an end of the plunger that forces fluid in the barrel through the needle as the plunger moves down through an injection stroke towards the needle opening; wherein the needle holder has self advancing release timing and is releasably attached with the barrel and a biasing member in the barrel biases the needle holder away from the needle opening, the needle holder having a resilient portion that engages with the hollow plunger to release the attachment between the needle holder and the barrel such that the needle holder is propelled by the biasing member to break through the deformable member and retract into the hollow plunger.

In accordance with the present invention there is also provided a syringe comprising: a barrel; a needle holder positioned inside the barrel that holds a hypodermic needle through a needle opening in the barrel; and a hollow plunger sealingly slidable within the barrel and having a deformable member across an end of the plunger that forces fluid in the barrel through the needle as the plunger moves down through an injection stroke towards the needle holder; wherein the needle holder is releasably attached with the barrel and a biasing member in the barrel biases the needle holder away from the needle opening, the needle holder includes a resilient portion having outer points and as the hollow plunger moves through the injection stroke the plunger engages with the resilient portion thereby moving the outer points up into the hollow plunger to release the attachment between the needle holder and the barrel such that the needle holder is propelled by the biasing member to break through the deformable member and retract into the hollow plunger.

Preferably, upon engagement with the plunger, the resilient portion is compressed such that the outer points move inwardly towards each other as well as up into the hollow plunger. The outer points of the resilient portion define a maximum width that is preferably larger than a minimum diameter at the end of the hollow plunger so that as the plunger draws down over the needle holder, the width of the resilient portion decreases. The minimum diameter at the end of the hollow plunger is defined by an inwardly tapering annular surface ending in an innermost edge.

The resilient portion of the needle holder is preferably defined by spaced projections that can resiliently move closer together. There are typically two spaced projection, each having outermost edges which are the outer points.

Preferably, protrusions carried at a base of the resilient portion engage with corresponding recesses in the barrel to attach the needle holder to the barrel.

The deformable member includes a lip that conforms around the needle holder as the hollow plunger slides over the needle holder to force fluid through the needle holder. The deformable member is an elastomeric sacrificial membrane stretched across the end of the hollow plunger.

In accordance with the present invention there is further still provided a method of automatic retraction of a hypodermic needle in a syringe including:
depressing a hollow plunger to sealingly slide within a barrel so that a deformable member across an end of the hollow plunger forces fluid in the barrel through the needle, wherein the needle is held in a needle holder releasably attached with the barrel;
sliding the end of the hollow plunger down over the needle holder to compress a resilient portion of the needle holder which causes outer points of the resilient portion to advance up toward the hollow plunger and the deformable member to stretch;
compressing the resilient portion until the engagement between the needle holder and the barrel is released;
propelling the needle holder and needle to rupture through the deformable member and into the hollow plunger.

The method preferably includes levering a protrusion on the resilient portion out of a recess in the barrel to release attachment between the needle holder and barrel by displacing the outer points up into the hollow plunger.

### BREIF DESCRIPTION OF THE DRAWINGS

Embodiments of the invention will be described with reference to the following drawings in which.
Figure 1 illustrates a single use syringe before operation in accordance with a preferred embodiment of the invention;
Figure 2 illustrates the syringe after it has been filled with fluid and ready for injection;
Figure 3 (a) illustrates the syringe toward the end of the injection stroke;
Figure 3 (b) is an enlarged view of the lower portion of the syringe of figure 3 (a);
Figure 4 (a) is a front elevation view of a preferred embodiment of the needle holder of the syringe in an open position;
Figure 4 (b) is a front elevation view of the needle holder in a closed position;
Figure 4 (c) is a side elevation of the needle holder of figure 4 (a);
Figure 4 (d) is a plan view of the needle holder of figure 4 (a) in an open position;
Figure 4 (e) is a plan view of the needle holder of figure 4 (a) in a closed position;
Figure 5 (a) illustrates the syringe with the plunger rod engaging with the needle holder;
Figure 5 (b) is an enlarged view of the lower portion of the syringe of figure 5 (a);
Figure 6 (a) illustrates the syringe with the plunger rod engaging further with the needle holder compared to figure (a);
Figure 6 (b) is an enlarged view of the lower portion of the syringe of figure 6 (a);
Figure 7 (a) illustrates the syringe with the plunger rod engaging still further with the needle holder compared to figure 6 (a) and at the end of the injection stroke;
Figure 7 (b) is an enlarged view of the lower portion of the syringe of figure 7 (a);
Figure 8 (a) illustrates the syringe with the plunger rod at the end of the injection stroke after the plunger seal has burst;
Figure 8 (b) is an enlarged view of the lower portion of the syringe of figure 8 (a);
Figure 9 (a) illustrates the syringe with the needle holder retracted;
Figure 9 (b) is an enlarged view of the upper portion of the syringe of figure 9 (a);
Figures 10 (a) and 10 (b) illustrate the syringe in an alternative embodiment in an exploded view and an assembled view;
Figure 11 (a) illustrates the syringe in yet another embodiment; Figure 11 (b) is an enlarged view of the needle holder of figure 11 (a);
Figure 12 illustrates a component of the barrel that is colour coded for needle gauge recognition of figure 11 (a);
Figure 13 (a) is a front elevation of alternative embodiment of the needle holder;
Figure 13 (b) is a side elevation of the needle holder of figure 13 (a);
Figure 13 (c) is a top elevation of the needle holder of figure 13 (a) in an open condition; and
Figure 13 (d) is a top elevation of the needle holder of figure 13 (a) in a closed condition.

### DETAILED DESCRIPTION OF PREFERED EMBODIMENTS

The drawings illustrate embodiments of a single use retractable syringe 100 that automatically retracts the needle with minimum force after the fluid within the syringe is dispensed so that the syringe can be safely disposed. This not only addresses needle stick injury by retracting the needle automatically with a minimum amount of force required for activation, but the syringe also has the ability to reduce the amount of dead or wasted space, namely the fluid left in the syringe after use, therefore minimising the chance of cross contamination by residual fluids. The syringe 100 has a barrel 1 that is largely tubular in shape and is formed to have a rearwardly open end 2 with a circular collar 5 to receive a plunger rod 6 and a forwardly end reduced in diameter to a central opening 102, namely closed end 3, that houses a needle holder 12. A hypodermic needle 14 is supported by the needle holder 12 and a spring 13 is compressed between the central opening 102 and needle holder 12.

The syringe is designed to have at least part of needle holder 12 and a hypodermic needle 14 protruding and exposed past the most forward part of the barrel at the closed end 3. The barrel also has opposing finger grips 4 to allow a user to depress the plunger rod 6 against the barrel 1. The barrel 1 can be made as a single piece from injection moulded plastics for example, UV resistant high clarity polypropylene, or any other material typically used to make syringe barrels.

The plunger rod 6 is largely tubular in shape and slides within barrel 1. It has a hollow retraction cavity 9 located inside the tubular form with an open end at a most forwardly portion 8 and has a closed end at rearwardly portion 7. The open end at the most forwardly section 8 is sealed by a plunger seal 11 attached around the outlet of the plunger rod 6.

Plunger seal 11 also spans across the open end at forward portion 8. It slidingly seals inside the barrel 1 to form a variable fluid chamber 16 between plunger seal 11 and the closed end 3 of the barrel 1. An operational grip or thumb pad 10 on the closed end of plunger rod 6 provides a surface for a user to depress against finger grips 4 on the barrel 1. The plunger rod can be made as a single piece from injection moulded plastics, such as a polypropylene. Talc powder could be added to the plastics to give plunger rod 6 strength and an opaque appearance. Alternatively, the plunger rod 6 could be made in multiple parts, as discussed further below.

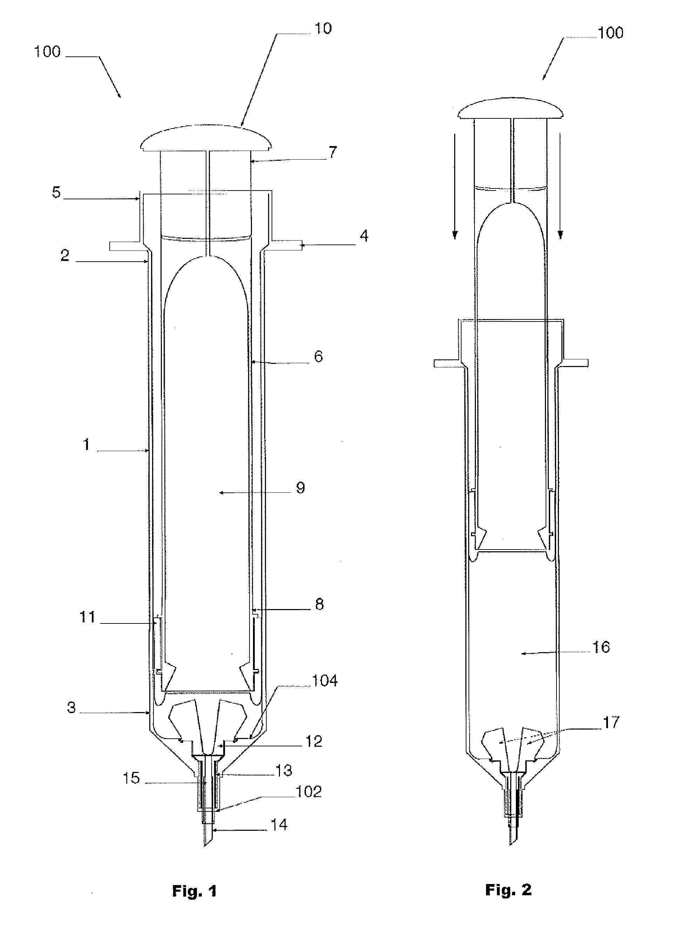

The syringe 100 functions similarly to standard syringes in that a user draws up the fluid into the variable chamber 16 by placing the hypodermic needle 14 into a fluid filled vial (not shown) and pulling back on the plunger rod 6 from its position shown in Figure 1 using the thumb pad 10. This causes a vacuum to form in the variable chamber 16 of the syringe and fluid is drawn up through the hypodermic needle 14, through a fluid path 15 of the needle holder 12 and into the variable fluid chamber 16 of the syringe barrel 1. Figure 2 illustrates fluid chamber filled with liquid and the syringe prepared for injection.

Before injection air bubbles in chamber 16 are removed by orientating the syringe with a needle directed upward so that gravity allows the fluid to flow to the plunger seal 11 and any air bubbles rise to the hypodermic needle 14. The user then expels any air bubbles by pushing the plunger rod 6 until only fluid remains in the variable fluid chamber 16. The user then places the hypodermic needle 14 into the patient and begins pushing on plunger rod 6 moving it towards the central opening 102 to expel the fluid from the variable fluid chamber 16.

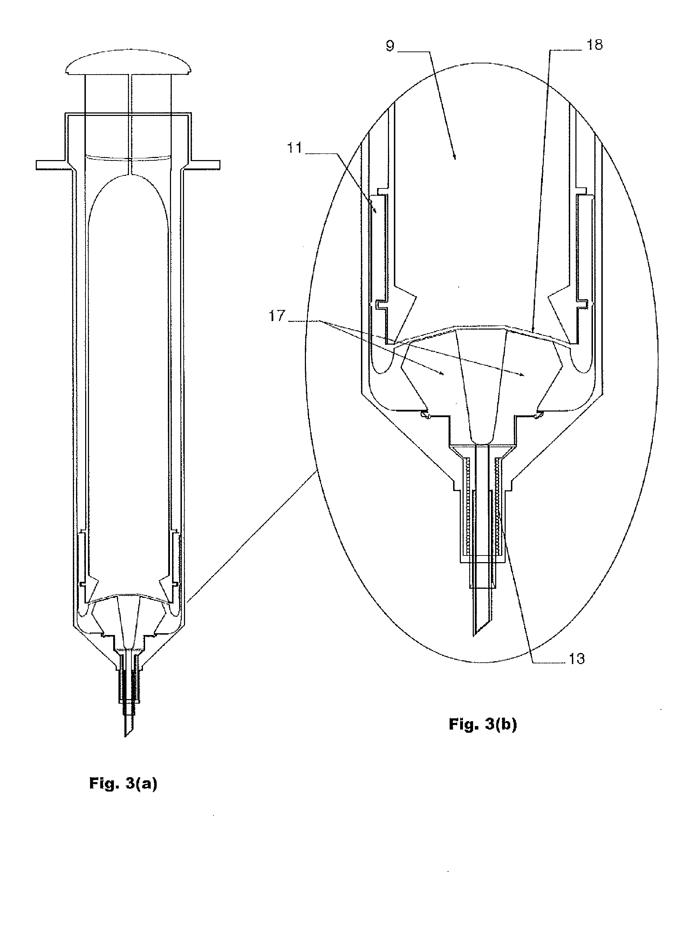

Figures 3 (a) and 3 (b) illustrate the plunger rod 6 approaching the end of the injection stroke and plunger seal 11 coming into contact with the needle holder 12. The needle holder 12 has extended resilient arms 17 that protrude upward into variable fluid chamber 16, and the plunger seal 11 at the open end of the plunger has a sacrificial deformable membrane stretched across the open end which starts to stretch and conform around arms 17 as plunger rod 6 is pushed further against needle holder 12.

The plunger seal 11 with its stretchable sacrificial membrane 18 can be moulded in one piece using an elastomeric material like Santoprene® which is commonly used in the syringe manufacturing industry and would preferably consist of a 181 type, 55 to 65 grade in order to obtain correct elasticity for best timing of plunger rod movement against the needle holder.

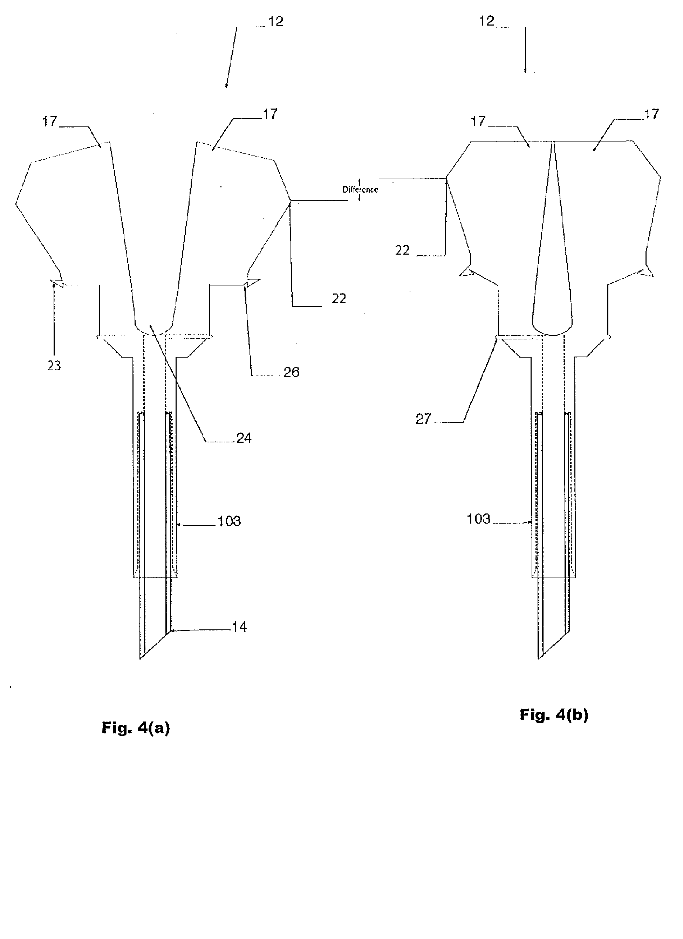

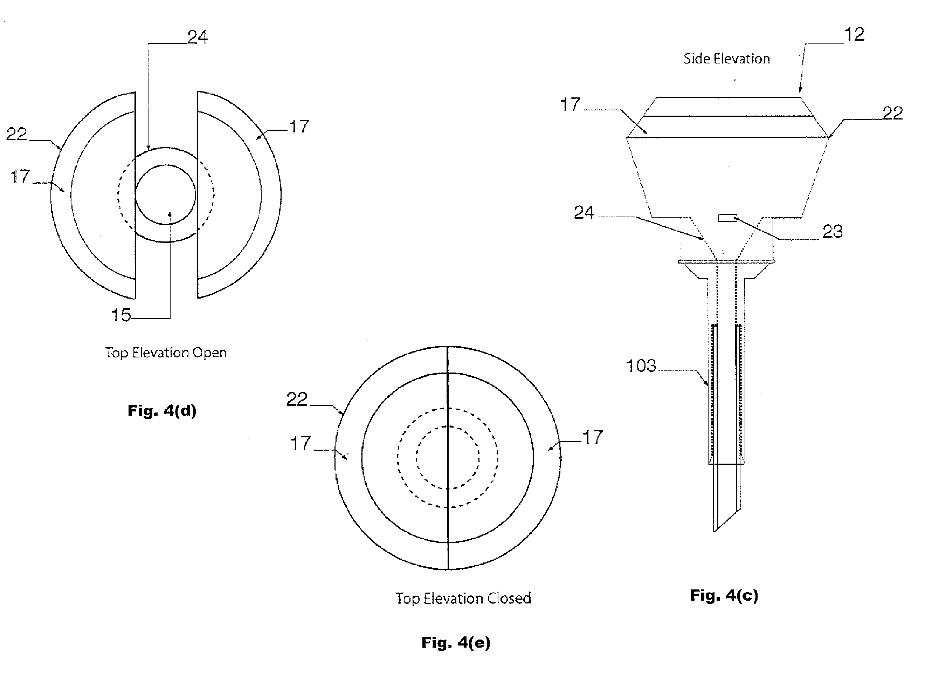

Figures 4 (a) and 4 (b) illustrate the needle holder 12 with Figures 4 (a), 4 (c) and 4 (d) showing the needle holder 12 in an open condition before retraction and Figures 4 (b) and 4 (e) showing the needle holder 12 in a closed condition during the retraction process. Needle holder 12 comprises a long hollow holding section 103, which receives the hypodermic needle 14, connected to a resilient portion, namely the two semi-circular resilient arms 17.

The needle holder 12 has scavenging ports 24 that are level or just below the most bottom part of the variable fluid chamber 16 of the barrel 1 maximising the amount of fluid that can be expelled before retraction, therefore reducing dead or waste space.

The needle holder is held in a first, non-retracted position in the closed end 3 of barrel 1 by small outward facing locking barbs 23, which are formed as part of the needle holder at the base of the resilient arms 17. The locking barbs 23 are configured to engage the back of the internal locking groove 25 formed in the closed end 3 of barrel 1 and are designed to withstand all rearward forces, namely forces by spring 13 directed rearwardly of barrel 1. The needle holder 12 would only succumb to these rearwardly forces when the locking barbs 23 have been levered past the internal locking groove 25 in the closed end 3 of barrel (1) and when the stretchable sacrificial membrane has burst. A raised internal lip inside the closed end 3 of barrel 1 could be used rather than a groove and to receive a corresponding recess on the needle holder. Resilient arms 17 are biased away from each other inherently by their material.

In use, the hollow plunger is depressed down over the resilient portion so that arms 17 are forced towards each other as shown in figures 4 (b) and 4 (e). Each arm 17 has an outer point, namely an apex 22, which is the outermost point across the resilient portion and which defines the maximum width of the resilient portion.

It can be seen, especially by figures 4 (a) and 4 (b) that there is a difference in apex height between the open and the closed positions of needle holder 12.

The plunger rod has an internal sloped protruding surface on the wall of the hollow cavity 9, namely a triggering mechanism or trigger surface 20. As plunger rod 6 is pushed in a downward direction toward the needle holder it starts to compress and close the needle holder's resilient arms 17. As this happens the apex 22 begins to automatically advance itself in an upwardly direction towards the plunger rod's trigger surface 20 and the apex of the trigger surface, namely trigger point 28. This process is defined as "self advancing needle holder timing".

Self advancing needle holder timing is where the apex 22 is urged to move up toward and into the hollow cavity 9 of plunger rod 6 (and specifically toward trigger point 28) as the plunger rod moves down and engages with the needle holder's resilient portion. This double acting movement between the resilient arms 17 and plunger rod 6 releases the engagement between the needle holder 12 and barrel 1 to allow spring 13 to push the needle holder through stretched membrane 18. Because the apex 22, which provides a leverage point for levering barbs 23 out of groove 25, moves towards the plunger rod, the effective length that the plunger rod travels to the end of the injection stroke is shorter than what it would be if apex 22 remained stationary.

This advancement of apex 22 has two distinct advantages: one is that the plunger rod travels a shorter distance to activate the retraction sequence so the engagement of the retraction mechanism is able to take place when the plunger rod is closer to the end of the injection stroke without compromising the force required to activate the syringe; the second advantage is that the retraction sequence is delayed to the last possible moment of the injection stroke and thus the fluid integrity of the syringe remains intact closer to the point of retraction, which minimises the chance of destabilizing the needle holder and the sealing arrangements by avoiding hydraulic dynamics that can cause premature activation and leaking by hydraulic forces.

It would be desirable for needle holder 12 to be made as a single part from a injection mouldable plastics such as polypropylene. A polystyrene could also be used because of its ability to be able to use a UV curable glue to attach a stainless hypodermic needle without having to prepare the surface of needle holder 12.

The mechanical advantage and mechanical leverage the needle holder has over the locking barbs 23 can be changed and increased by moving the distance of apex 22 further away from locking barbs 23 than that illustrated in the drawings. This would give greater mechanical advantage by the needle holder resilient arms 17 over the locking barbs 23 and thereby reduce activation force without compromising the force required to hold the needle holder 12 against the bias of the compressed spring 13 and other forces, such as pushing the needle into the thick rubber bung of a medical vial.

Locking barbs 23 could be designed to fracture or break by placing a weakening groove 26 on the forward side of the locking barbs 23 in order to further reduce the activation force and also not allowing the needle holder to be refitted into a non-retracting position after use therefore making syringe 100 self-destructing and non-reusable.

Because the needle holder 12 is held in place in the closed end 3 of barrel 1 by mechanical means it is not adversely affected by the hydraulic dynamics that cause premature activation, and namely by hydraulic forces that are generated by depressing the plunger rod 6 and forcing the fluid in the variable fluid chamber 16 into the smaller diameter of the needle holder fluid path 15.

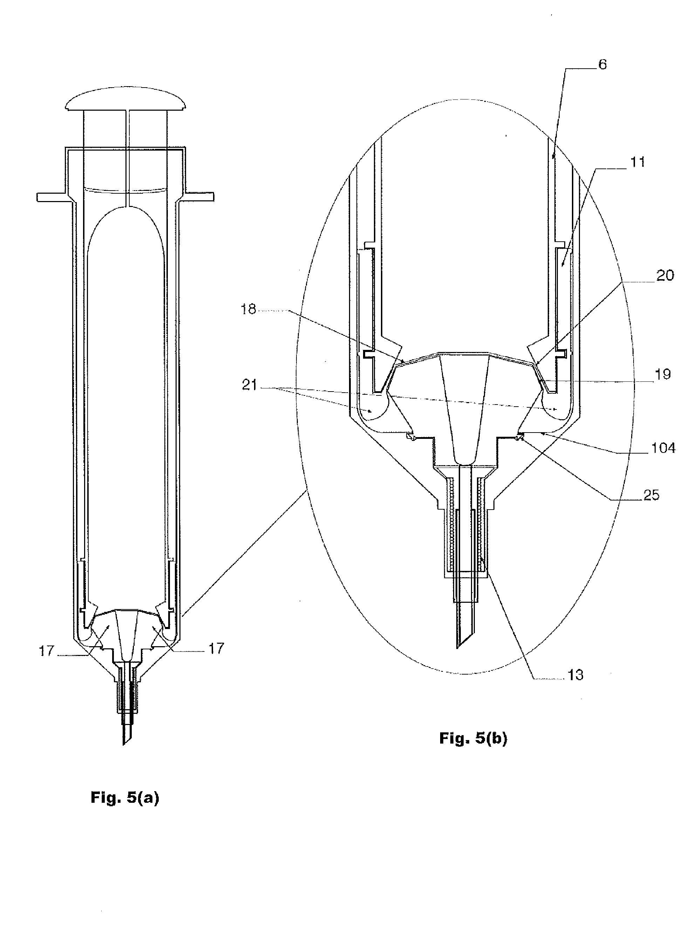

Figure 5 (a) and 5 (b) illustrate the plunger rod 6 travelling further towards the end of the injection stroke. Outer inclined surfaces 19 of the resilient arms 17 above apex 22 begin to force the stretchable sacrificial membrane 18 of the plunger seal 11 against the trigger surface 20 as the plunger rod continues to travel downwardly towards the central opening 102. A few things begin to happen simultaneously: the trigger surface 20 forces the resilient arms 17 to close together. At the same time a forward part 21 of the plunger seal is designed to roll inwardly towards the needle holder while the seal is being stretched.

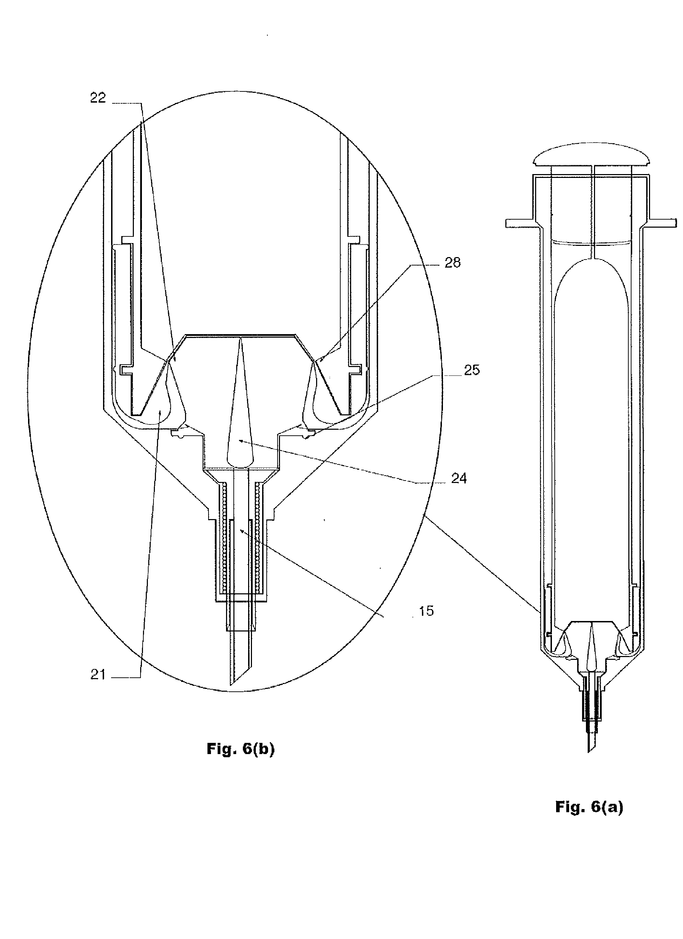

Figures 6 (a) and 6 (b) illustrate the plunger rod 6 has been depressed and is coming to the end of the injection stroke. The needle holder locking barbs 23 have now been levered out of the internal locking groove 25 due to the compression of resilient arms 17, which are forced together by the trigger surface 20 as the plunger rod reaches the end of the injection stroke. The resilient arms' apex 22 and trigger point 28 of the trigger surface 20 are now level and the resilient arms 17 have now closed together and the only thing holding the needle holder 12 against the closed end of the barrel 1 is the sacrificial membrane 18 that has been stretched to almost its limit. The syringe is mechanically in automatic retracting mode.

Meanwhile, the forward part of the plunger seal 21 of plunger seal 11 is rolls inwardly towards the needle holder 12. As the top of the membrane is stretched over resilient arms 17, forward part 21 conforms to the shape of the needle holder and channels the last of the fluid in the variable fluid chamber 16 into the fluid scavenging ports 24 and out through the needle holder fluid path 15.

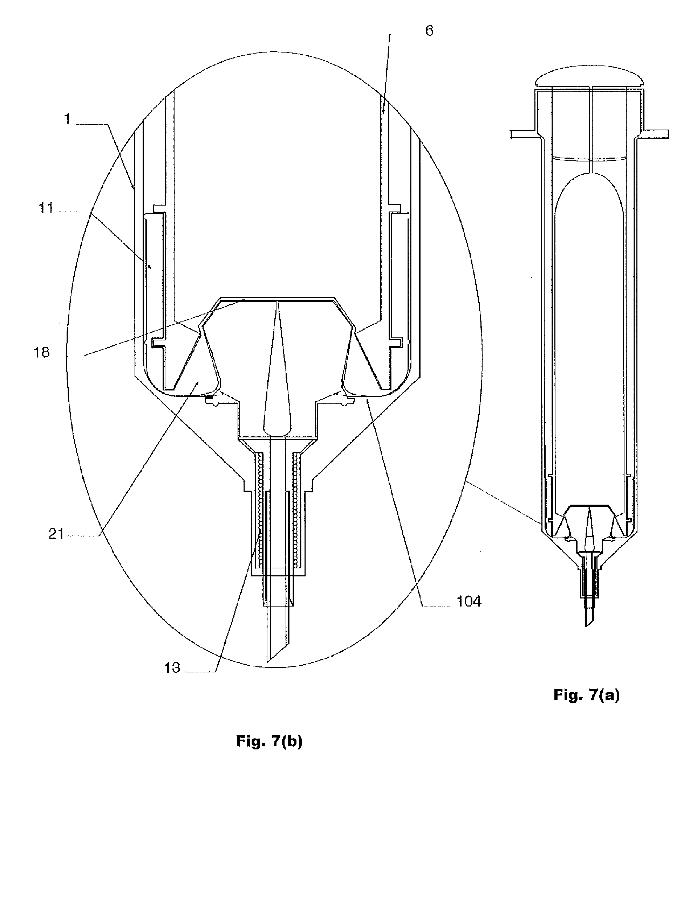

Figure 7 (a) and 7 (b) show the plunger rod 6 with the plunger seal 11 coming to the end of the injection stroke and forward part 21 of the plunger seal draws even closer to the needle holder 12. The front part of the plunger seal which covers the end walls of the forward section 8 of plunger rod 6 comes to rest on a front wall 104 of the closed end 3 of barrel 1 thereby expelling all of fluid from the variable fluid chamber as the stretchable sacrificial membrane 18 is now stretched to the point that it can no longer withstand the biasing force of the compressed spring 13.

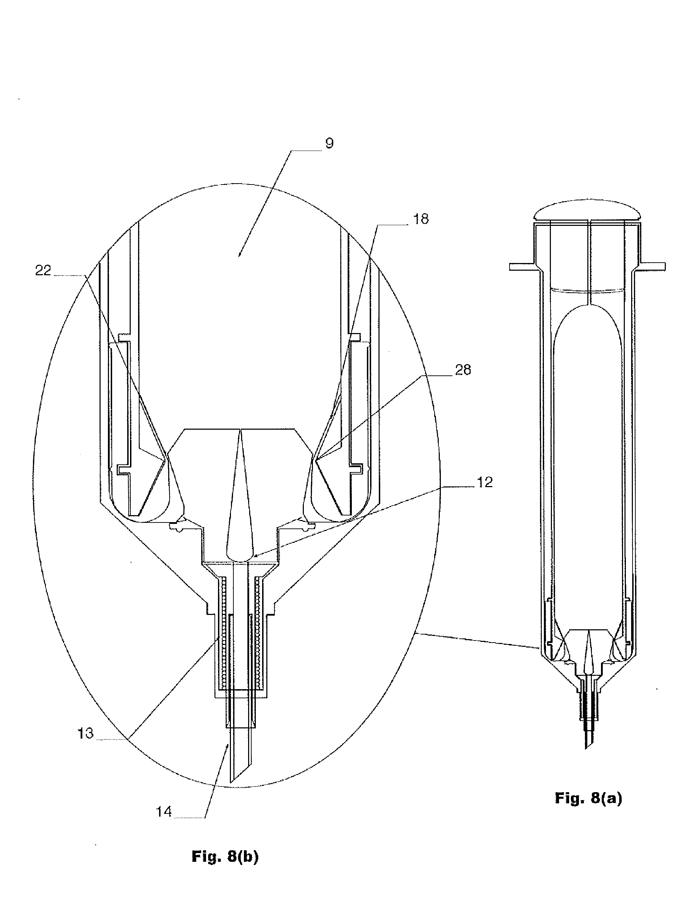

Figures 8 (a) and 8 (b) illustrate the point where the stretchable sacrificial membrane 18 has burst and the needle holder apex 22 is above the trigger point 28. This causes the needle holder needle holder 12 with the hypodermic needle 14 attached to it to automatically pass through into the retraction cavity 9 propelled by the spring 13.

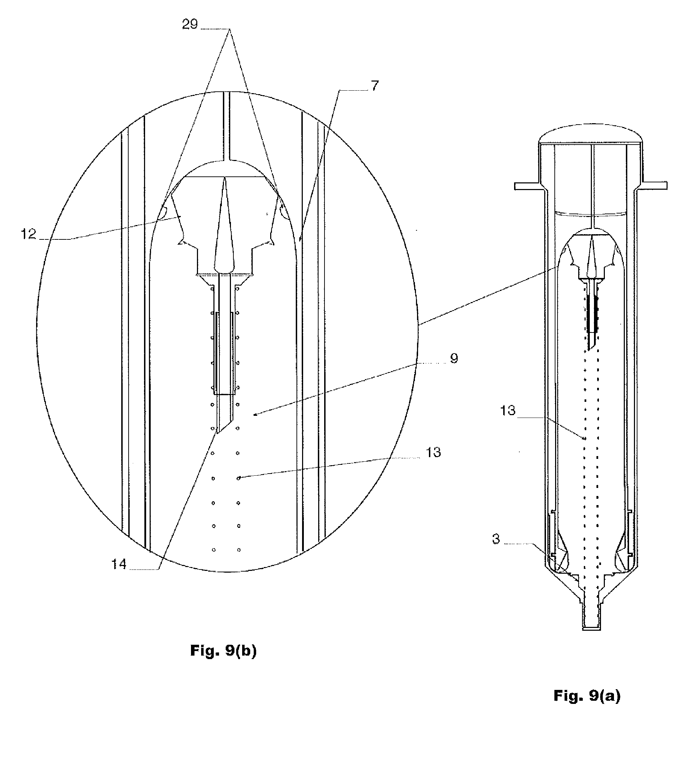

Figures 9 (a) and 9 (b) illustrate the needle holder 12 and hypodermic needle 14 retracted into the hollow plunge rod and retained in the rearward section 7 of the plunger rod 6 by expanded spring 13. In a preferred embodiment, and as illustrated in the drawings, retraction cavity restraining stops 29 are mounted inside of the rearward 5 section 7. The needle holder 12 can pass by the stops 29 but is prevented from returning so that the hypodermic needle 14 remains safely inside the retraction cavity 9 leaving only a small portion of the spring 13 as the only part of the retraction mechanism left inside the closed end 3 of the barrel 1 after retraction the hypodermic needle therefore no longer poses a threat to anyone and is unable to be reused.

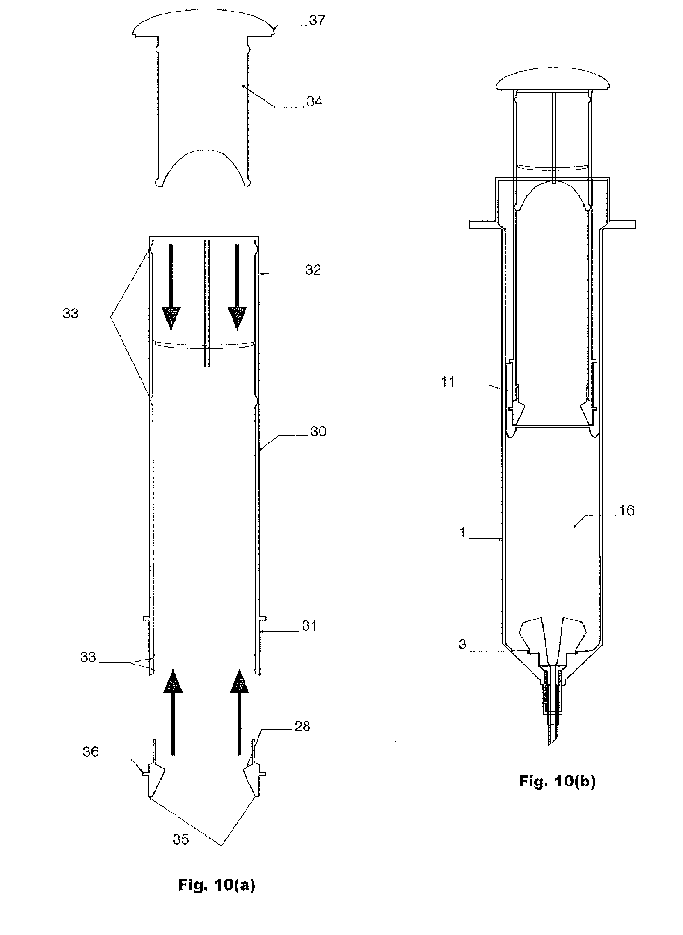

Figures 10 (a) and 10 (b) are illustrations of an alternative embodiment of the single-use retractable syringe. While it is desirable to have as few parts as possible in order to have a syringe that is reliable and cost effective to mass produce, some components can include multiple parts, particularly if this enhances features of the syringe at a minimal cost and without affecting the mechanical reliability of the syringe.

For example, and as illustrated in Figures 10 (a) and 10 (b), the plunger rod 6 is made in three pieces and consists of a plunger rod barrel, a detachable trigger piece 35 and a detachable operational grip 34 with thumb pad. Plunger rod barrel 30 is largely tubular in shape and could be made as a single piece from injection moulded plastics such as a polypropylene. Talc powder or the like could be added to the polypropylene to give the barrel 30 added strength and an opaque appearance. The plunger rod barrel 30 is similar in configuration to plunger rod 6 of the previous embodiment but includes internal locking grooves or barbs 33 located inside a forward section 31 and a rearward section 32 of barrel 30 to receive by engagement the detachable internal trigger piece 35 at the forward section 31, and the operational grip 34 at the and retained in the rearward section 7 of the plunger rod 6 by expanded spring 13. In a preferred embodiment, and as illustrated in the drawings, retraction cavity restraining stops 29 are mounted inside of the rearward section 7. The needle holder 12 can pass by the stops 29 but is prevented from returning so that the hypodermic needle 14 remains safely inside the retraction cavity 9 leaving only a small portion of the spring 13 as the only part of the retraction mechanism left inside the closed end 3 of the barrel 1 after retraction the hypodermic needle therefore no longer poses a threat to anyone and is unable to be reused.

Figures 10 (a) and 10 (b) are illustrations of an alternative embodiment of the single-use retractable syringe. While it is desirable to have as few parts as possible in order to have a syringe that is reliable and cost effective to mass produce, some components can include multiple parts, particularly if this enhances features of the syringe at a minimal cost and without affecting the mechanical reliability of the syringe.

For example, and as illustrated in Figures 10 (a) and 10 (b), the plunger rod 6 is made in three pieces and consists of a plunger rod barrel, a detachable trigger piece 35 and a detachable operational grip 34 with thumb pad. Plunger rod barrel 30 is largely tubular in shape and could be made as a single piece from injection moulded plastics such as a polypropylene. Talc powder or the like could be added to the polypropylene to give the barrel 30 added strength and an opaque appearance. The plunger rod barrel 30 is similar in configuration to plunger rod 6 of the previous embodiment but includes internal locking grooves or barbs 33 located inside a forward section 31 and a rearward section 32 of barrel 30 to receive by engagement the detachable internal trigger piece 35 at the forward section 31, and the operational grip 34 at the rearward section 32. Aside from engagement with the barbs or grooves, the trigger piece 35 and grip 34 could be also attached by press fit or gluing.

The trigger piece 35 is circular in shape and has a conical sloping interior that comes to a peak at an apex 28. The piece also has an annular shoulder 36 around the outside to engage with a corresponding circular groove in the plunger seal 11. A similar engagement between the trigger surface 20 and seal 11 exists for the previous embodiment of the syringe. The trigger piece 35 resists any rearward movement during the retraction process as the needle holder 12 propels past, trigger piece could be made as a single piece from injection mouldable plastics such as a polypropylene. An oil based type of injectable plastics such as Aseatoll® would be desirable because it has a high level of hardness and its oily characteristics would aid in lubrication between the trigger piece 35 and the stretchable sacrificial membrane 18 during the retraction process.

The detachable operational grip 34 has a forward section 36 and a rearward section 37. The forward section is configured to be attached to the rearward section 32 of plunger rod barrel 30 either by press fit, gluing or engaging with the locking barbs or groves 33. The rearward section 37 is configured so that pressure applied to it by a user's thumb is evenly distributed which causes less pain to the user. One advantage of having a detachable operational grip or thumb pad rather than it being moulded with the rest of the plunger rod is the ability to replace the grip with one of a different colour. A procedure by the International Organization for Standardization (ISO) identifies different needle gauges by colour, for example a 21 gauge needle is identified by the colour green, in order that the connect needle can be identified quickly without the need to read the package.

This becomes extremely helpful in emergencies when there is no time to read packages or where the syringe has been previously taken out of the package.

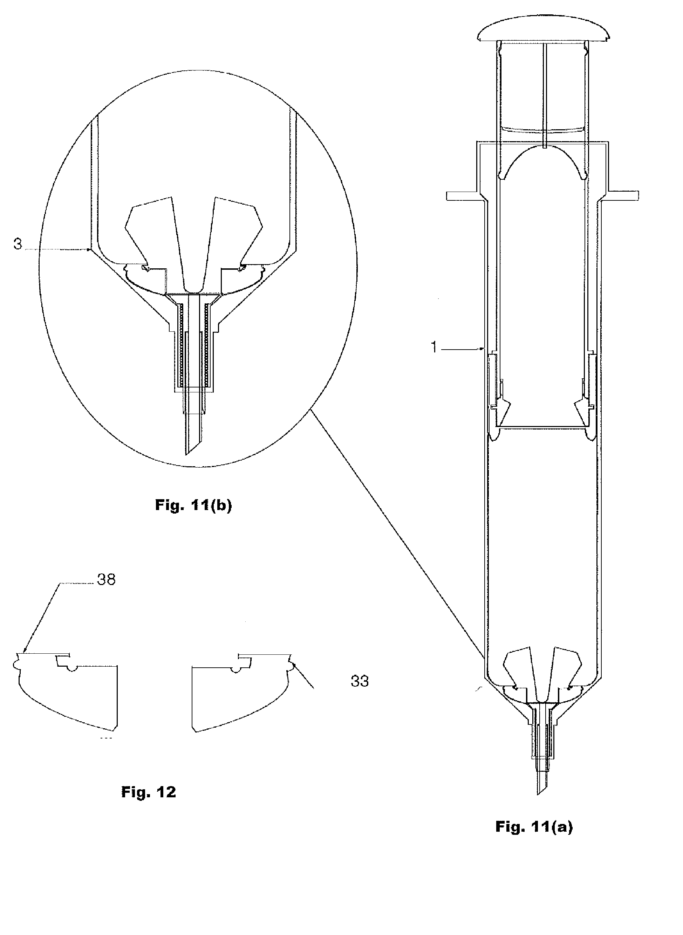

Figures 11 (a) and 11 (b) illustrate a single use retractable syringe in yet another embodiment. In this embodiment the internal locking grooves at the closed end 3 of the barrel are carried by an annular insert 38 which is attached permanently or detachably to the closed end 3 of syringe barrel 1 by way of either gluing or engagement between locking barbs or grooves 33 as shown in Figures 11 (a) and 11 (b). Figures 11 (a) and 11 (b) show insert 38 being coloured. This would be helpful for the same reasons given above. The advantage of having both the grip and insert coloured is that the device can be colour coded at both ends. The coloured insert 38 can be made as a single piece from injection mouldable plastics for example polypropylene.

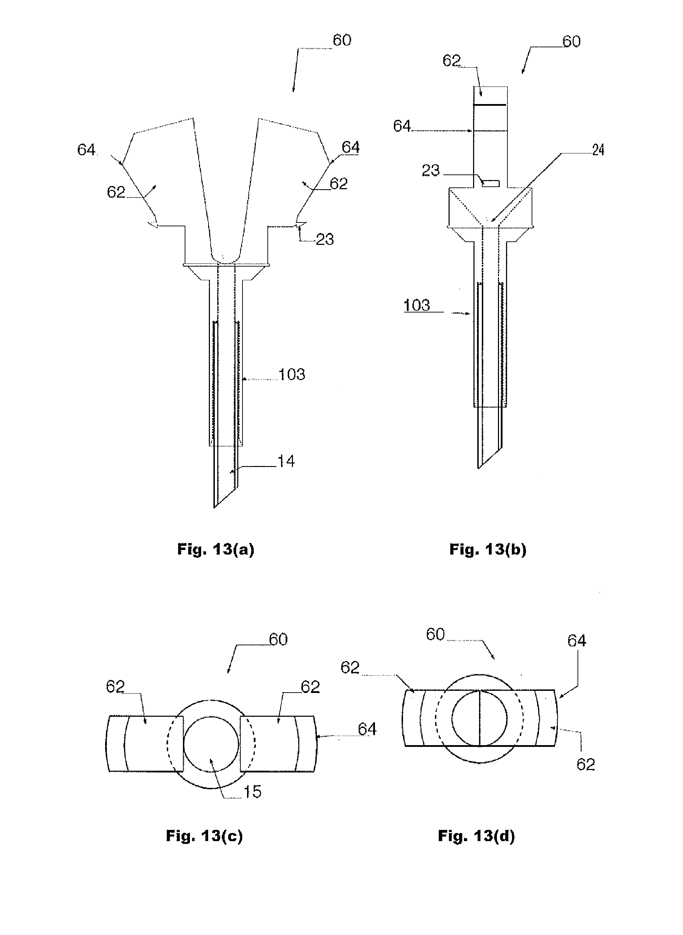

Figures 13 (a) to 13 (d) illustrate another embodiment of a needle holder 60. In this embodiment the needle holding section 103 of the needle holder 60 is the same as the needle holder 12 of the first embodiment. The resilient aspect is however different.

In side profile the needle holders 12 and 60 are similar. However, rather than having broad semi-circular resilient arms as in needle holder 12, needle holder 60 has narrower arms 62, as best shown in the plan views of Figures 13 (c) and 13 (d). Profile arms 64 also have an elbow defining the outwardly most point at apex 64 where the diameter of the needle holder 60 is largest, and which reacts against trigger surface 20, which has a smaller diameter, to compress resilient arms 62 and release needle holder 60 from engagement with the barrel 1.

The syringe provides a means of a safe syringe injection with automatic and total needle retraction without causing the recipient additional pain, for example by depressing the plunger further than the end of the injection stroke in order to activate the retraction mechanism, which is required by some known syringes. Cross contamination by splatter is minimised and the syringe cannot be tampered with for re-use.

The self advancing release timing feature of the syringe means there is a smooth transition from the end of the injection stroke and into needle retraction mode. Retraction mode starts before the plunger rod finishes the injection stroke by the needle holder's resilient arms beginning to advance in the opposite direction to the injection stroke and toward the trigger surface of the plunger rod. The timing of the plunger rod components and needle holder components is such that when all the fluid has been expelled from the variable chamber, the resilient arms lever out the needle holder's connection with the barrel so that the needle holder is no longer restrained by the barrel and is subjected to the full force of the spring to rupture through the stretched membrane and retract into the plunger rod.

The foregoing embodiments are illustrative only of the principles of the invention, and various modifications and changes will readily occur to those skilled in the art. The invention is capable of being practiced and carried out in various ways and in other embodiments. It is also to be understood that the terminology employed herein is for the purpose of description and should not be regarded as limiting.

In the present specification and claims, the word `comprising' and its derivatives including 'comprises' and 'comprise' include each of the stated integers but does not exclude the inclusion of one or more further integers.

Reference throughout this specification to 'one embodiment' or 'an embodiment' means that a particular feature, structure, or characteristic described in connection with the embodiment is included in at least one embodiment of the present invention. Thus, the appearance of the phrases 'in one embodiment' or 'in an embodiment' in various places throughout this specification are not necessarily all referring to the same embodiment. Furthermore, the particular features, structures, or characteristics may be combined in any suitable manner in one or more combinations.

In compliance with the statute, the invention has been described in language more or less specific to structural or methodical features. It is to be understood that the invention is not limited to specific features shown or described since the means herein described comprises preferred forms of putting the invention into effect. The invention is, therefore, claimed in any of its forms or modifications within the proper scope of the appended claims appropriately interpreted by those skilled in the art.

## Claims

1. A syringe comprising:
a barrel (1);
a needle holder (12) positioned inside the barrel (1) that holds a hypodermic needle (14) through a needle opening (102) in the barrel (1);
a hollow plunger (6) sealingly slidable within the barrel (1) and having a deformable member (18) across an end of the hollow plunger (6) that forces fluid in the barrel (1) through the hypodermic needle (14) as the hollow plunger (6) moves down through an injection stroke towards the needle opening (102); and
a biasing member (13) positioned in the barrel (1) and adapted to bias the needle holder (12) away from the needle opening (102) toward the hollow plunger (6),
**characterized in that**
the needle holder (12) is releasably attached to the barrel (1) and includes a resilient portion (17) that engages with the hollow plunger (6) to release the attachment between the needle holder (12) and the barrel (1) such that the needle holder (12) is propelled by the biasing member (13) to break through the deformable member (18) and retract with the hypodermic needle (14) into the hollow plunger (6).

2. The syringe according to claim 1, wherein the resilient portion (17) includes outer points (22, 22) that advance in the opposite direction to the hollow plunger (6) as the hollow plunger (6) moves through the injection stroke and engages with the resilient portion (17) to release the attachment between the needle holder (12) and the barrel (1).

3. The syringe according to claim 2, wherein upon engagement with the hollow plunger (6), the resilient portion (17) is compressed such that the outer points (22, 22) move inwardly towards each other as well as up into the hollow plunger (6).

4. The syringe according to claim 2 or claim 3, wherein the outer points (22, 22) of the resilient portion (17) define a maximum width that is larger than a minimum diameter at the end of the hollow plunger (6) so that as the hollow plunger (6) draws down over the needle holder (12) the width of the resilient portion (17) decreases.

5. The syringe according to claim 4, wherein the minimum diameter at the end of the hollow plunger (6) is defined by an inwardly tapering annular surface (20) ending in an innermost edge (28).

6. The syringe according to any one of claims 1 to 5, wherein the resilient portion (17) of the needle holder (12) is defined by spaced projections (17, 17) that can resiliently move closer together.

7. The syringe according to claim 6, wherein there are two spaced projections (17, 17) each having outermost edges which are the outer points (22, 22).

8. The syringe according to any one of claims 1 to 7, wherein the needle holder (12) is releasably attached to the barrel (1) by a protrusion provided at a base of the resilient portion (17) that engages with a corresponding recess provided in the barrel (1).

9. The syringe according to claim 8, wherein the protrusion is one or more outwardly locking barbs (23) formed as part of the needle holder (12) at the base of the resilient portion (17) and configured to engage with the corresponding recess comprising one or more internal locking grooves (25) formed in the barrel (1).

10. The syringe according to any one of claims 1 to 9, wherein the deformable member (18) includes a lip (21) that conforms around the needle holder (12) as the hollow plunger (6) slides over the needle holder (12) to force fluid through the needle holder (12).

11. The syringe according to any one of claims 1 to 10, wherein the deformable member (18) is an elastomeric sacrificial membrane stretched across the end of the hollow plunger (6).

12. The syringe according to any one of claims 1 to 11, wherein the needle holder (12) includes a scavenger port (26) adapted to maximize the quantity of fluid expelled before retraction of the needle holder (12) and the hypodermic needle (14).

13. The syringe according to any one of claims 1 to 12, wherein the hollow plunger (6) includes inwardly projecting restraining stops (29) located towards a rearward section (7) of the hollow plunger (6) and adapted to retain the needle holder (12) and the hypodermic needle (14) within the hollow plunger (6) after retraction of the needle holder (12) and the hypodermic needle (14).

14. A method of automatic retraction of a hypodermic needle in a syringe as defined in claim 1,
**characterized in that**
the method includes the steps of:
(a) depressing a hollow plunger (6) which sealingly slides within a barrel (1) so that a deformable member (18) located across an end of the hollow plunger (6) forces fluid in the barrel (1) through a hypodermic needle (14), wherein the hypodermic needle (14) is held in a needle holder (12) releasably attached to the barrel (1);
(b) sliding the end of the hollow plunger (6) down over the needle holder (12) to compress a resilient portion (17) of the needle holder (12) thereby causing outer points (22, 22) of the resilient portion (17) to advance up toward the hollow plunger (6) and causing the deformable member (18) to stretch;
(c) compressing the resilient portion (17) until the attachment between the needle holder (12) and the barrel (1) is released; and
(d) propelling the needle holder (12) and the hypodermic needle (14) to rupture through the deformable member (18) and retract into the hollow plunger (6).

15. The method of automatic retraction according to claim 14, wherein in step (c) includes levering a protrusion located on a base of the resilient portion (17) out of a recess located in the barrel (1) to release the attachment between the needle holder (12) and the barrel (1) by displacing the outer points (22, 22) up into the hollow plunger (6).
